(19) Europäisches Patentamt / European Patent Office / Office européen des brevets

(11) EP 4 475 207 A1

(12) **EUROPEAN PATENT APPLICATION**

(43) Date of publication:
**11.12.2024 Bulletin 2024/50**

(21) Application number: **24179930.3**

(22) Date of filing: **04.06.2024**

(51) International Patent Classification (IPC):
***H01M 4/13*** *(2010.01)* ***H01M 10/0525*** *(2010.01)*
***H01M 10/0567*** *(2010.01)*

(52) Cooperative Patent Classification (CPC):
**H01M 10/0525; H01M 4/13; H01M 4/525; H01M 10/052; H01M 10/0567; H01M 10/4235**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.06.2023 KR 20230072444**

(71) Applicant: **Samsung SDI Co., Ltd.**
**Yongin-si, Gyeonggi-do 17084 (KR)**

(72) Inventors:
- **Park, Hongryeol**
  **17084 Yongin-si, Gyeonggi-do (KR)**
- **Choi, Hyunbong**
  **17084 Yongin-si, Gyeonggi-do (KR)**
- **Park, Injun**
  **17084 Yongin-si, Gyeonggi-do (KR)**
- **Park, Sangwoo**
  **17084 Yongin-si, Gyeonggi-do (KR)**
- **Kim, Sohee**
  **17084 Yongin-si, Gyeonggi-do (KR)**
- **Yang, Yeji**
  **17084 Yongin-si, Gyeonggi-do (KR)**
- **Kim, Sanghoon**
  **17084 Yongin-si, Gyeonggi-do (KR)**

(74) Representative: **Gulde & Partner**
**Patent- und Rechtsanwaltskanzlei mbB**
**Wallstraße 58/59**
**10179 Berlin (DE)**

(54) **RECHARGEABLE LITHIUM BATTERY**

(57) A rechargeable lithium battery includes: a positive electrode including a cobalt-free positive electrode active material with a layered structure; a negative electrode; and an electrolyte including an additive represented by Chemical Formula 1:

Chemical Formula 1

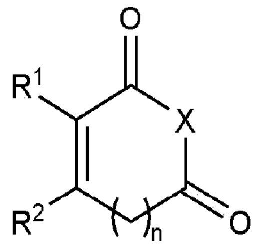

.

EP 4 475 207 A1

## Description

## BACKGROUND

### 1. Field

One or more embodiments of the present disclosure relate to a rechargeable lithium battery.

### 2. Description of the Related Art

A rechargeable lithium battery may be recharged and has energy density that is three or more times higher per unit weight as a comparable lead storage battery, nickel-cadmium battery, nickel hydrogen battery, nickel zinc battery and/or the like. The rechargeable lithium battery may be also charged at a relatively high rate and thus, is commercially manufactured for a laptop, a cell phone, an electric tool, an electric bike, and/or the like, and research on improvement of additional energy density has been actively conducted or pursued.

The rechargeable lithium battery is formed or realized by injecting an electrolyte into an electrode assembly including a positive electrode including a positive electrode active material and a negative electrode including a negative electrode active material.

One of the recent development interests and directions for a rechargeable lithium battery is to improve high-temperature charge/discharge and storage characteristics while improving room-temperature charge/discharge characteristics. In general, rechargeable lithium batteries have issues in that their resistance increases and/or their cycle-life decreases as they are repeatedly charged and discharged at room temperature. These issues becomes more severe if utilizing a cobalt-free positive electrode active material and/or storing the rechargeable lithium batteries at high tem peratures.

## SUMMARY

The invention is defined by the appended claims. The description that follows is subjected to this limitation. Any disclosure lying outside the scope of said claims is only intended for illustrative as well as comparative purposes. One or more aspects of embodiments of the present disclosure are directed toward a rechargeable lithium battery in which an increase in resistance is suppressed, maintained, or reduced at room temperature and/or at high temperature and cycle-life characteristics are improved while utilizing a cobalt-free positive electrode active material. Additional aspects will be set forth in part in the description which follows and, in part, will be apparent from the description, or may be learned by practice of the presented embodiments of the disclosure.

According to one or more embodiments of the present disclosure, a rechargeable lithium battery includes: a positive electrode including a cobalt-free positive electrode active material with a layered structure; a negative electrode; and an electrolyte including an additive represented by Chemical Formula 1:

**Chemical Formula 1**

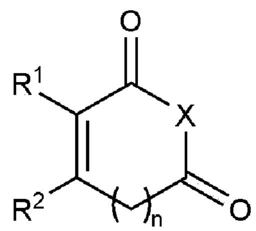

,

wherein, in Chemical Formula 1,

X is O or S;
$R^1$ and $R^2$ are each independently hydrogen, an unsubstituted C1 to C10 alkyl group, or are linked to each other to form (or provide) a C6 to C20 cyclic hydrocarbon ring or aromatic ring; and
n is an integer from 0 to 3.

In the rechargeable lithium battery of one or more embodiments, by utilizing a cobalt-free positive electrode active material and utilizing a cyclic acid anhydride-based compound as an electrolyte additive, an increase in resistance

at room temperature and/or at high temperature may be suppressed, mainlined, or reduced and cycle-life characteristics thereof may be improved.

## BRIEF DESCRIPTION OF THE DRAWINGS

The above and other aspects, features, and advantages of certain embodiments of the present disclosure will be more apparent from the following description taken in conjunction with the accompanying drawings.
FIGs. 1-3 are each a schematic view showing a rechargeable lithium battery according to one or more embodiments of the present disclosure.

## DETAILED DESCRIPTION

The present disclosure may be modified in many alternate forms, and thus specific embodiments will be exemplified in the drawing and described in more detail.
Hereinafter, a rechargeable lithium battery according to one or more embodiments will be described in more detail with reference to the accompanied drawings.

### Rechargeable Lithium Battery

In one or more embodiments of the present disclosure, a rechargeable lithium battery includes: a positive electrode including a cobalt-free positive electrode active material with a layered structure; a negative electrode; and an electrolyte including an additive represented by Chemical Formula 1:

**Chemical Formula 1**

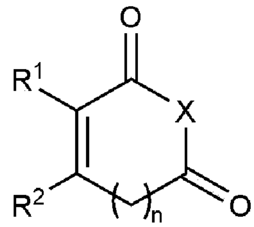
,

wherein, in Chemical Formula 1,

X are oxygen (O) or sulfur (S);
$R^1$ and $R^2$ are each independently hydrogen, an unsubstituted C1 to C10 alkyl group, or are linked to each other to form (or provide) a C6 to C20 cyclic hydrocarbon ring (e.g., aliphatic ring) or aromatic ring; and
n is an integer from 0 to 3.

### Positive Electrode Active Material

Recently, in response to cobalt depletion and rising costs, research on "cobalt-free positive electrode active materials" is underway and/or urgently pursued. The cobalt-free positive electrode active material refers to a positive electrode active material with a layered structure composed of nickel, manganese, and/or the like as main components and does not contain cobalt in the positive electrode active material composition. In one or more embodiments, the rechargeable lithium battery utilizes a cobalt-free positive electrode active material. In present disclosure, "not include a or any 'component'," "exclude a or any 'component'", "'component'-free", and/or the like refers to that the "component" not being added, selected, or utilized as a component in a composition, but the "component" of less than a suitable amount may still be included due to other impurities and/or external factors.
The cobalt-free positive electrode active material may include at least one type or kind of lithium composite oxide represented by Chemical Formula 2.

**Chemical Formula 2** $Li_aNi_xMn_yM^1_zM^2_wO_{2\pm b}X_c$

In Chemical Formula 2,

$0.5 \leq a < 1.8$, $0 \leq b \leq 0.1$, $0 \leq c \leq 0.1$, $0 \leq w < 0.1$, $0.6 \leq x < 1.0$, $0 < y < 0.4$, $0 < z < 0.1$, $w + x + y + z = 1$,
$M^1$ and $M^2$ may each independently be one or more elements selected from among aluminum (Al), magnesium (Mg), titanium (Ti), zirconium (Zr), chromium (Cr), strontium (Sr), vanadium (V), boron (B), tungsten (W), molybdenum (Mo), niobium (Nb), silicon (Si), barium (Ba), calcium (Ca), cerium (Ce), and iron(Fe), and X may be one or more elements selected from among sulfur (S), fluorine (F), phosphorous (P), and chlorine (Cl).

In one or more embodiments, Chemical Formula 2 may be represented by Chemical Formula 2-1.

**Chemical Formula 2-1** $Li_aNi_{x1}Mn_{y1}Al_{z1}M^2_{w1}O_{2\pm b}X_c$

In Chemical Formula 2-1,

$0.5 \leq a < 1.8$, $0 \leq b \leq 0.1$, $0 \leq c \leq 0.1$, $0 \leq w1 < 0.1$, $0.6 \leq x1 < 1.0$, $0 < y1 < 0.4$, $0 < z1 < 0.1$, $w1 + x1 + y1 + z1 = 1$,
$M^2$ may be one or more elements selected from among Mg, Ti, Zr, Cr, Sr, V, B, W, Mo, Nb, Si, Ba, Ca, Ce, and Fe, and X may be one or more elements selected from among S, F, P, and Cl.

In some embodiments, in Chemical Formula 2-1,
$0.7 \leq x1 < 1.0$, $0 < y1 < 0.3$, $0 < z1 < 0.1$, or $0.8 \leq x1 < 1.0$, $0 < y1 < 0.2$, $0 < z1 < 0.1$.

If the transition metal(s) eluted from the positive electrode active material is reduced on the surface of the negative electrode during operation of the rechargeable lithium battery, it may weaken the surface of the negative electrode, causing increased battery resistance and reduced cycle-life, and in the long term, lithium dendrites may be formed, causing a short circuit between the positive electrode and the negative electrode.

The cobalt-free positive electrode active material has high structural instability due to the absence of cobalt, so there is a high probability that nickel and/or manganese will be eluted during operation of the rechargeable lithium battery. Additionally, this issue may become more severe at high temperatures.

**Additive**

In one or more embodiments, the additive is an aromatic cyclic anhydride-based compound represented by Chemical Formula 1.

The cyclic acid anhydride-based compound (Chemical Formula 1) is reduced and decomposed on the surface of the negative electrode during operation of the rechargeable lithium battery and thus forms a stable solid electrolyte interface (SEI). Accordingly, if an electrolyte prepared by adding the cyclic acid anhydride-based compound (Chemical Formula 1) is utilized, even though the cobalt-free positive electrode active material with a layered structure is utilized, the stable SEI film may improve cycle-life characteristics as well as protect the surface of the negative electrode and suppress or reduce an increase in resistance during operation of the rechargeable lithium battery at room temperature and/or a high temperature.

The description of Chemical Formula 1 representing the additive is as follows.

In one or more embodiments, X may be O.

$R^1$ and $R^2$ may each independently be hydrogen or a methyl group, or may be linked to each other to form (or provide) a cyclohexane ring.
n may be 0 or 1.

Non-limiting representative examples of the additive represented by Chemical Formula 1 may be as follows:

**Chemical Formula 1-1**

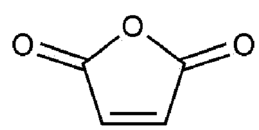

Chemical Formula 1-2

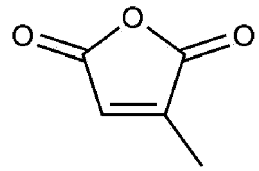

Chemical Formula 1-3

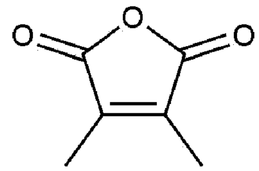

Chemical Formula 1-4

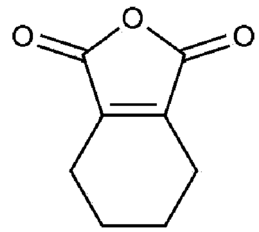

Chemical Formula 1-5

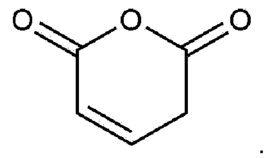

In one or more embodiments, the additive may be included in an amount of about 0.05 to about 3 wt%, about 0.05 to about 2 wt%, or about 0.05 to about 1 wt%, based on a total weight, 100 wt%, of the electrolyte. Within this range, the cyclic acid anhydride-based compound (Chemical Formula 1) may be reduced and decomposed on the surface of the negative electrode to form (or provide) a stable SEI film.

**Non-aqueous Organic Solvent**

The electrolyte may further include a non-aqueous organic solvent.

The non-aqueous organic solvent serves as a medium for transmitting ions taking part in the electrochemical reaction of a battery.

The non-aqueous organic solvent may be a carbonate-based, ester-based, ether-based, ketone-based, or alcohol-based solvent, an aprotic solvent, or a (e.g., any suitable) combination thereof.

The carbonate-based solvent may include dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and/or the like. The ester-based solvent may include methyl acetate, ethyl acetate, n-propyl acetate, dimethylacetate, methylpropionate, ethylpropionate, decanolide, mevalonolactone, valerolactone, caprolactone, and/or the like

The ether-based solvent may include dibutyl ether, tetraglyme, diglyme, dimethoxyethane, 2-methyltetrahydrofuran, 2,5-dimethyltetrahydrofuran, tetrahydrofuran, and/or the like. The ketone-based solvent may include cyclohexanone and/or the like. The alcohol-based solvent may include ethyl alcohol, isopropyl alcohol, and/or the like, and the aprotic solvent may include nitriles such as R-CN (wherein R is a C2 to C20 linear, branched, or cyclic hydrocarbon

group, and may include a double bond, an aromatic ring, or an ether group), amides such as dimethylformamide, dioxolanes such as 1,3-dioxolane or 1,4-dioxolane, sulfolanes, and/or the like.

The non-aqueous organic solvent may be utilized alone or in combination of two or more thereof, and if utilized in combination of two or more, a mixing ratio may be appropriately adjusted according to the desired or suitable battery performance, which is well understood by those skilled in the art.

In some embodiments, if utilizing a carbonate-based solvent, a cyclic carbonate and a chain carbonate may be mixed and utilized, and the cyclic carbonate and the chain carbonate may be mixed at a volume ratio of about 1:1 to about 1:9.

In one or more embodiments, the non-aqueous organic solvent may further include an aromatic hydrocarbon-based organic solvent. For example, in some embodiments, a carbonate-based solvent and an aromatic hydrocarbon-based organic solvent may be mixed and utilized in a volume ratio of about 1:1 to about 30:1.

In one or more embodiments, the electrolyte may further include vinylethyl carbonate, vinylene carbonate, or an ethylene carbonate-based compound to improve battery cycle-life.

Non-limiting representative examples of the ethylene carbonate-based compound may include fluoroethylene carbonate, difluoroethylene carbonate, chloroethylene carbonate, dichloroethylene carbonate, bromoethylene carbonate, dibromoethylene carbonate, nitroethylene carbonate, and cyanoethylene carbonate.

**Lithium Salt**

The electrolyte may further include a lithium salt.

The lithium salt dissolved in the non-aqueous organic solvent supplies lithium ions in a battery, enables a basic operation of a rechargeable lithium battery, and improves transportation of the lithium ions between positive and negative electrodes. Non-limiting examples of the lithium salt may include at least one selected from among $LiPF_6$, $LiBF_4$, $LiSbF_6$, $LiAsF_6$, $LiClO_4$, $LiAlO_2$, $LiAlCl_4$, $LiPO_2F_2$, LiCl, LiI, $LiN(SO_3C_2F_5)_2$, $Li(FSO_2)_2N$ (lithium bis(fluorosulfonyl)imide, LiFSI), $LiC_4F_9SO_3$, $LiN(C_xF_{2x+1}SO_2)(C_yF_{2y+1}SO_2)$ (wherein x and y are each an integer of 1 to 20), lithium trifluoromethane sulfonate, lithium tetrafluoroethanesulfonate, lithium difluorobis(oxalato)phosphate (LiDFBOP), and lithium bis(oxalato) borate (LiBOB).

A concentration of lithium salt may be within the range of about 0.1 M to about 2.0 M. When the concentration of lithium salt is within the above range, the electrolyte has appropriate or suitable ionic conductivity and viscosity, and thus excellent or suitable performance may be achieved and lithium ions may move effectively.

**Positive Electrode**

The positive electrode for a rechargeable lithium battery may include a positive electrode current collector and a positive electrode active material layer on the positive electrode current collector. The positive electrode active material layer may include a positive electrode active material and may further include a binder and/or a conductive material.

A content of the positive electrode active material may be about 90 wt% to about 99.5 wt% based on a total weight, 100 wt%, of the positive electrode active material layer.

In one or more embodiments, the positive electrode active material layer may further include a binder and a conductive material. At these embodiments, content of the binder and the conductive material may each be about 0.5 wt% to about 5 wt%, based on a total weight, 100 wt%, of the positive electrode active material layer.

The binder serves to attach the positive electrode active material particles well to each other and also to attach the positive electrode active material well to the positive electrode current collector. Examples of the binder may include polyvinyl alcohol, carboxymethyl cellulose, hydroxypropyl cellulose, diacetyl cellulose, polyvinylchloride, carboxylated polyvinylchloride, polyvinylfluoride, a polymer including ethylene oxide, polyvinylpyrrolidone, polyurethane, polytetrafluoroethylene, polyvinylidene fluoride, polyethylene, polypropylene, a styrene-butadiene rubber, a (meth)acrylated styrene-butadiene rubber, an epoxy resin, a (meth)acrylic resin, a polyester resin, nylon, and/or the like, as non-limiting examples.

The conductive material may be utilized to impart conductivity (e.g., electrical conductivity) to the positive electrode. Any material that does not cause chemical change (e.g., does not cause an undesirable chemical change in the rechargeable lithium battery) and conducts electrons may be utilized in the battery. Non-limiting examples of the conductive material may include: a carbon-based material such as natural graphite, artificial graphite, carbon black, acetylene black, Ketjen black, a carbon fiber, a carbon nanofiber, and carbon nanotube; a metal-based material containing copper, nickel, aluminum, silver, and/or the like, in a form of a metal powder or a metal fiber; a conductive polymer such as a polyphenylene derivative; or a mixture thereof.

In one or more embodiments, aluminum (Al) may be utilized as the positive electrode current collector, but embodiments of the present disclosure are not limited thereto.

## Negative Electrode Active Material

A negative electrode active material may be a material that reversibly intercalates/deintercalates lithium ions, a lithium metal, a lithium metal alloy, a material capable of doping and dedoping lithium, or a transition metal oxide.

The material that reversibly intercalates/deintercalates lithium ions may include a carbon-based negative electrode active material, for example, crystalline carbon, amorphous carbon, and/or a (e.g., any suitable) combination thereof. The crystalline carbon may be graphite such as non-shaped (e.g., irregularly shaped), sheet-shaped, flake-shaped, sphere-shaped, or fiber-shaped natural graphite or artificial graphite. The amorphous carbon may be a soft carbon, a hard carbon, a mesophase pitch carbonization product, calcined coke, and/or the like.

The lithium metal alloy may include lithium and a metal selected from among sodium (Na), potassium (K), rubidium (Rb), cesium (Cs), francium (Fr), beryllium (Be), magnesium (Mg), calcium (Ca), strontium (Sr), silicon (Si), antimony (Sb), lead (Pb), indium (In), zinc (Zn), barium (Ba), radium (Ra), germanium (Ge), aluminum (Al), and tin (Sn).

The material capable of doping/dedoping lithium may be a Si-based negative electrode active material or a Sn-based negative electrode active material. The Si-based negative electrode active material may include silicon, a silicon-carbon composite, $SiO_x$ ($0 < x \leq 2$), a Si-Q alloy (where Q is selected from among an alkali metal, an alkaline-earth metal, a Group 13 element, a Group 14 element (excluding Si), a Group 15 element, a Group 16 element, a transition metal, a rare earth element, and a (e.g., any suitable) combination thereof, for example magnesium (Mg), calcium (Ca), strontium (Sr), barium (Ba), radium (Ra), scandium (Sc), yttrium (Y), titanium (Ti), zirconium (Zr), hafnium (Hf), rutherfordium (Rf), vanadium (V), niobium (Nb), tantalum (Ta), dubnium (Db), chromium (Cr), molybdenum (Mo), tungsten (W), seaborgium (Sg), technetium (Tc), rhenium (Re), bohrium (Bh), iron (Fe), lead (Pb), ruthenium (Ru), osmium (Os), hassium (Hs), rhodium (Rh), iridium (Ir), palladium (Pd), platinum (Pt), copper (Cu), silver (Ag), gold (Au), zinc (Zn), cadmium (Cd), boron (B), aluminum (Al), gallium (Ga), tin (Sn), indium (In), thallium (Tl), germanium (Ge), phosphorous (P), arsenic (As), antimony (Sb), bismuth (Bi), sulfur (S), selenium (Se), tellurium (Te), polonium (Po), and/or a (e.g., any suitable) combination thereof). The Sn-based negative electrode active material may include Sn, $SnO_2$, a Sn-based alloy, and/or a (e.g., any suitable) combination thereof.

The silicon-carbon composite may be a composite of silicon and amorphous carbon. An average particle diameter ($D_{50}$) of the silicon-carbon composite particle may be for example about 0.5 microns ($\mu$m) to about 20 $\mu$m. According to one or more embodiments, the silicon-carbon composite may be in a form of silicon particles and amorphous carbon coated on the surface of the silicon particles. For example, in some embodiments, the silicon-carbon composite may include a secondary particle (core) in which primary silicon particles are assembled, and an amorphous carbon coating layer (shell) on the surface of the secondary particle. The amorphous carbon may also be between the primary silicon particles, and, for example, the primary silicon particles may be coated with the amorphous carbon. The secondary particle may exist dispersed in an amorphous carbon matrix.

The silicon-carbon composite may further include crystalline carbon. For example, in one or more embodiments, the silicon-carbon composite may include a core including crystalline carbon and silicon particles and an amorphous carbon coating layer on a surface of the core. The crystalline carbon may be artificial graphite, natural graphite, or a (e.g., any suitable) combination thereof. The amorphous carbon may include soft carbon or hard carbon, a mesophase pitch carbonized product, and/or calcined coke.

In one or more embodiments, if the silicon-carbon composite includes silicon and amorphous carbon, a content of silicon may be about 10 wt% to about 50 wt%, and a content of amorphous carbon may be about 50 wt% to about 90 wt% based on a total weight, 100 wt%, of the silicon-carbon composite. In one or more embodiments, if the silicon-carbon composite includes silicon, amorphous carbon, and crystalline carbon, a content of silicon may be about 10 wt% to about 50 wt%, and a content of crystalline carbon may be about 10 wt% to about 70 wt%, and a content of amorphous carbon may be about 20 wt% to about 40 wt%, based on a total weight, 100 wt%, of the silicon-carbon composite.

In one or more embodiments, a thickness of the amorphous carbon coating layer may be about 5 nanometers (nm) to about 100 nm. An average particle diameter ($D_{50}$) of the silicon particles (primary particles) may be about 10 nm to about 1 $\mu$m, or about 10 nm to about 200 nm. The silicon particles may exist as silicon alone, in the form of a silicon alloy, or in an oxidized form. The oxidized form of silicon may be represented by $SiO_x$ ($0 < x \leq 2$). In one or more embodiments, an atomic content ratio of Si:O, which indicates a degree of oxidation, may be 99:1 to 33:67. As utilized herein, if a definition is not otherwise provided, an average particle diameter (D50) indicates a particle diameter of a particle where an accumulated volume is about 50 volume% in a particle distribution.

In some embodiments, the Si-based negative electrode active material or the Sn-based negative electrode active material may be utilized in combination with a carbon-based negative electrode active material. If utilizing a mixture of a Si-based negative electrode active material or a Sn-based negative electrode active material and a carbon-based negative electrode active material, a mixing ratio thereof may be about 1:99 to about 90:10 by weight.

**Negative Electrode**

The negative electrode for a rechargeable lithium battery may include a negative electrode current collector and a negative electrode active material layer on the negative electrode current collector. The negative electrode active material layer includes a negative electrode active material and may further include a binder and/or a conductive material.
A content of the negative electrode active material may be about 95 wt% to about 99.5 wt% based on a total weight, 100 wt%, of the negative electrode active material layer. A content of the binder may be about 0.5 wt% to about 5 wt% based on the total weight, 100 wt%, of the negative electrode active material layer. A content of the conductive material may be about 0.5 wt% to about 5 wt% based on the total weight, 100 wt%, of the negative electrode active material layer. For example, in some embodiments, the negative electrode active material layer may include about 90 wt% to about 99 wt% of the negative electrode active material, about 0.5 wt% to about 5 wt% of the binder, and about 0.5 wt% to about 5 wt% of the conductive material.
The binder may serve to attach the negative electrode active material particles well to each other and also to attach the negative electrode active material well to the negative electrode current collector. The binder may include a non-aqueous (e.g., water-insoluble) binder, an aqueous (e.g., water-soluble) binder, a dry binder, and/or a (e.g., any suitable) combination thereof.
The non-aqueous binder may include polyvinyl chloride, carboxylated polyvinyl chloride, polyvinyl fluoride, an ethylene propylene copolymer, polystyrene, polyurethane, polytetrafluoroethylene, polyvinylidene fluoride, polyethylene, polypropylene, polyamideimide, polyimide, and/or a (e.g., any suitable) combination thereof.
The aqueous binder may be selected from among a styrene-butadiene rubber, a (meth)acrylated styrene-butadiene rubber, a (meth)acrylonitrile-butadiene rubber, (meth)acrylic rubber, a butyl rubber, a fluoro rubber, polyethylene oxide, polyvinylpyrrolidone, polyepichlorohydrin, polyphosphazene, poly(meth)acrylonitrile, an ethylene propylene diene copolymer, polyvinylpyridine, chlorosulfonated polyethylene, latex, a polyester resin, a (meth)acrylic resin, a phenol resin, an epoxy resin, polyvinyl alcohol, and/or a (e.g., any suitable) combination thereof.
When an aqueous binder is utilized as the binder of the negative electrode , the binder may further include a cellulose-based compound capable of imparting viscosity. The cellulose-based compound may include one or more of carboxylmethyl cellulose, hydroxypropylmethyl cellulose, methyl cellulose, or alkali metal salts thereof. The alkali metal may be Na, K, or Li.
The dry binder may be a polymer material capable of being fiberized, and may be, for example, polytetrafluoroethylene, polyvinylidene fluoride, polyvinylidene fluoride-hexafluoropropylene copolymer, polyethylene oxide, or a (e.g., any suitable) combination thereof.
In one or more embodiments, the conductive material may be included to provide and/or improve electrode conductivity, and any electrically conductive material may be utilized as a conductive material unless it causes a chemical change. Non-limiting examples of the conductive material may be: a carbon-based material such as natural graphite, artificial graphite, carbon black, acetylene black, Ketjen black, a carbon fiber, a carbon nanofiber, a carbon nanotube, and/or the like; a metal-based material such as copper, nickel, aluminum silver, and/or the like in a form of a metal powder or a metal fiber; a conductive polymer such as a polyphenylene derivative; or a mixture thereof.
The negative electrode current collector may include one selected from among a copper foil, a nickel foil, a stainless steel foil, a titanium foil, a nickel foam, a copper foam, a polymer substrate coated with a conductive metal, and/or a (e.g., any suitable) combination thereof, but embodiments of the present disclosure are not limited thereto.

**Separator**

Depending on the type or kind of the rechargeable lithium battery, a separator may be present between the positive electrode and the negative electrode. The separator may include polyethylene, polypropylene, polyvinylidene fluoride, or a multilayer film of two or more layers thereof, and a mixed multilayer film such as a polyethylene/polypropylene two-layer separator, a polyethylene/polypropylene/polyethylene three-layer separator, a polypropylene/polyethylene/polypropylene three-layer separator, and/or the like.
The separator may include a porous substrate and a coating layer including an organic material, an inorganic material, and/or a (e.g., any suitable) combination thereof on a surface (e.g., one or both (e.g., simultaneously) surfaces or sides (e.g., opposite surfaces or sides)) of the porous substrate.
The porous substrate may be a polymer film formed of any one selected from among polymer polyolefin such as polyethylene and/or polypropylene, polyester such as polyethylene terephthalate and/or polybutylene terephthalate, polyacetal, polyamide, polyimide, polycarbonate, polyether ketone, polyarylether ketone, polyether ketone, polyetherimide, polyamideimide, polybenzimidazole, polyethersulfone, polyphenylene oxide, a cyclic olefin copolymer, polyphenylene sulfide, polyethylene naphthalate, a glass fiber, and polytetrafluoroethylene (e.g., TEFLON), or a copolymer thereof, or a mixture of two or more thereof.
The porous substrate may have a thickness of about 1 μm to about 40 μm, for example, about 1 μm to about

30 μm, about 1 μm to about 20 μm, about 5 μm to about 15 μm, or about 10 μm to about 15 μm.

In some embodiments, the organic material may include a (meth)acryl-based copolymer including a first structural unit derived from (meth)acrylamide and a second structural unit including at least one of a structural unit derived from (meth)acrylic acid or (meth)acrylate, or a structural unit derived from (meth)acrylamidosulfonic acid or a salt thereof.

The inorganic material may include inorganic particles selected from among $Al_2O_3$, $SiO_2$, $TiO_2$, $SnO_2$, $CeO_2$, MgO, NiO, CaO, GaO, ZnO, $ZrO_2$, $Y_2O_3$, SrTiOs, BaTiOs, $Mg(OH)_2$, boehmite, and/or a (e.g., any suitable) combination thereof, but embodiments of the present disclosure are not limited thereto. An average particle diameter ($D_{50}$) of the inorganic particles may be about 1 nm to about 2000 nm, for example, about 100 nm to about 1000 nm, or about 100 nm to about 700 nm.

In one or more embodiments, the organic material and the inorganic material may be mixed in one coating layer, or a coating layer including an organic material and a coating layer including an inorganic material may be stacked.

A thickness of the coating layer may be about 0.5 μm to about 20 μm, for example, about 1 μm to about 10 μm, or about 1 μm to about 5 μm.

**Rechargeable Lithium Battery**

The rechargeable lithium battery may be classified into cylindrical, prismatic, pouch, or coin-type or kind batteries, and/or the like depending on a shape thereof. FIGs. 1 to 3 are each a schematic view showing a rechargeable lithium battery according to one or more embodiments. Referring to FIGs. 1 to 3, a rechargeable lithium battery 100 may include an electrode assembly 40 with a separator 30 between a positive electrode 10 and a negative electrode 20, and a case 50 in which the electrode assembly 40 is housed. The positive electrode 10, negative electrode 20, and separator 30 may be impregnated with an electrolyte. The rechargeable lithium battery 100 may include a sealing member 60 that seals the case 50 as shown in FIG. 1, or as shown in FIG. 3, it may further include an electrode tab 70 that serves as an electrical path for guiding the current generated in the electrode assembly 40 to the outside.

Hereinafter, examples and comparative examples of the present disclosure will be described. The following examples is only examples of the present disclosure, and the present disclosure is not limited to the following examples.

**Preparation of Electrolyte**

**Example 1**

An electrolyte was prepared by mixing ethylene carbonate (EC), ethylmethyl carbonate (EMC), and dimethyl carbonate (DMC) in a volume ratio of 2:4:4 to prepare a mixed non-aqueous organic solvent, dissolving 1.5 M $LiPF_6$ therein, and adding 0.05 wt% of an additive represented by Chemical Formula 1-1.

**Chemical Formula 1-1**

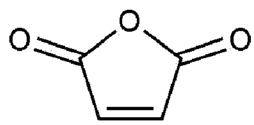

Herein, in the composition of an electrolyte, a content "wt%" of an additive is based on a total amount, 100 wt%, of the electrolyte (a lithium salt + a non-aqueous organic solvent + an additive).

**Example 2**

An electrolyte was prepared in substantially the same manner as in Example 1 except that the additive represented by Chemical Formula 1-1 was added in an amount of 0.1 wt%.

**Example 3**

An electrolyte was prepared in substantially the same manner as in Example 1 except that the additive represented by Chemical Formula 1-1 was added in an amount of 0.5 wt%.

**Example 4**

An electrolyte was prepared in substantially the same manner as in Example 1 except that the additive represented by Chemical Formula 1-1 was added in an amount of 1.0 wt%.

**Example 5**

An electrolyte was prepared in substantially the same manner as in Example 1 except that the additive represented by Chemical Formula 1-1 was added in an amount of 3.0 wt%.

**Example 6**

An electrolyte was prepared in substantially the same manner as in Example 1 except that 0.5 wt% of an additive represented by Chemical Formula 1-2 was added instead of the additive represented by Chemical Formula 1-1.

## Chemical Formula 1-2

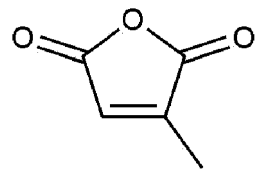

**Example 7**

An electrolyte was prepared in substantially the same manner as in Example 1 except that 0.5 wt% of an additive represented by Chemical Formula 1-3 was added instead of the additive represented by Chemical Formula 1-1.

## Chemical Formula 1-3

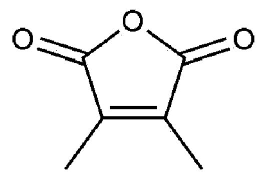

**Example 8**

An electrolyte was prepared in substantially the same manner as in Example 1 except that 0.5 wt% of an additive represented by Chemical Formula 1-4 was added instead of the additive represented by Chemical Formula 1-1.

## Chemical Formula 1-4

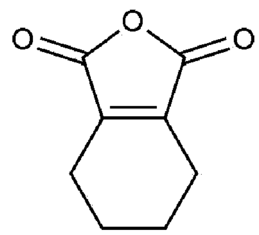

**Example 9**

An electrolyte was prepared in substantially the same manner as in Example 1 except that 0.5 wt% of an additive represented by Chemical Formula 1-5 was added instead of the additive represented by Chemical Formula 1-1.

## Chemical Formula 1-5

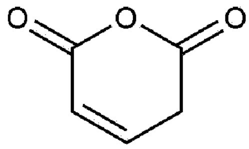

.

### Comparative Example 1 (Ref.)

An electrolyte was prepared in substantially the same manner as in Example 1 except that no additive was added at all.

### Comparative Example 2

An electrolyte was prepared in substantially the same manner as in Example 1 except that 0.5 wt% of an additive represented by Chemical Formula A was added instead of the additive represented by Chemical Formula 1-1.

## Chemical Formula A

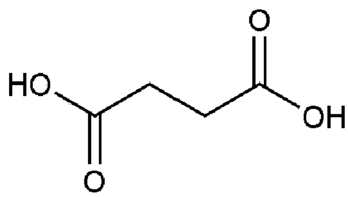

### Manufacture of Rechargeable Lithium Battery Cells

$LiNi_{0.75}Mn_{0.23}Al_{0.02}O_2$ as a positive electrode active material, polyvinylidene fluoride as a binder, and acetylene black as a conductive material were mixed respectively in a weight ratio of 96:3:1, and then, dispersed in N-methyl pyrrolidone to prepare a positive electrode active material slurry.

The positive electrode active material slurry was coated on a 15 μm-thick Al foil, dried at 100 °C, and then pressed to manufacture a positive electrode.

A mixture of artificial graphite and a Si-C composite in a weight ratio of 93:7 was prepared as a negative electrode active material, and the negative electrode active material, a styrene-butadiene rubber binder, and carboxylmethyl cellulose in a weight ratio of 98:1:1 were dispersed in distilled water to prepare a negative electrode active material slurry.

The Si-C composite included a core including artificial graphite and silicon particles and carbonyl pitch coated on the surface of the core.

The negative electrode active material slurry was coated on a 10 μm-thick Cu foil, dried at 100 °C, and then pressed to manufacture a negative electrode.

An electrode assembly was manufactured by assembling the positive electrode, the negative electrode, and a separator made of polyethylene with a thickness of 10 μm, and the electrolytes according to Examples 1 to 9 and Comparative Examples 1 and 2 were injected, respectively, to manufacture corresponding rechargeable lithium battery cells.

### Evaluation Examples

The rechargeable lithium battery cells manufactured by respectively utilizing the electrolytes according to Examples 1 to 9 and Comparative Examples 1 and 2 were each evaluated as follows, and the results are shown in Tables 1 to 3.

### Evaluation 1: Evaluation of Room-temperature Charging and Discharging Cycle Characteristics

Each of the rechargeable lithium battery cells according to Examples 1 to 9 and Comparative Examples 1 and

2 was charged and discharged under the following conditions to evaluate cycle characteristics, and the results are shown in Table 1.

The cells were 800 cycles charged and discharged under 0.33 C charge (CC/CV, 4.45 V, 0.025 C Cut-off) /1.0 C discharge (CC, 2.5 V Cut-off) conditions at 25 °C to measure capacity retention and a change in direct current internal resistance (DC-IR).

The capacity retention was calculated according to Equation 1, and the DC internal resistance change rate was calculated according to Equation 2 based on a voltage changed while discharged by applying a current of SOC 50 C for 30 seconds.

Capacity retention rate = (discharge capacity after 800 cycles / discharge capacity after 1 cycle) * 100 equation 1

DC internal resistance change rate = {(DC-IR after 800 cycles) / (DC-IR after 1 cycle)} * 100 equation 2

Table 1

| | Capacity retention rate @25 °C, 800th cyc. (%) | Initial DC-IR (DC-IR after 1 cycle) @25 °C (mΩ) | DC-IR after 800 cy @25 °C (mΩ) | DC-IR change rate after 800 cyc. @25 °C (%) |
|---|---|---|---|---|
| Example 1 | 76.4 | 21.24 | 25.51 | 120.1 |
| Example 2 | 78.8 | 21.72 | 25.70 | 118.3 |
| Example 3 | 72.7 | 22.00 | 28.60 | 130.0 |
| Example 4 | 70.0 | 23.13 | 31.32 | 135.4 |
| Example 5 | 64.1 | 23.92 | 32.53 | 136.0 |
| Example 6 | 75.5 | 22.18 | 26.79 | 120.8 |
| Example 7 | 73.2 | 22.34 | 27.75 | 124.2 |
| Example 8 | 70.7 | 22.26 | 27.07 | 121.6 |
| Example 9 | 65.5 | 23.05 | 28.74 | 124.7 |
| Comparative Example 1 | 55.4 | 20.97 | 30.76 | 146.7 |
| Comparative Example 2 | 61.0 | 22.90 | 31.83 | 139.0 |

Referring to Table 1, when the additive according to one or more embodiments is utilized at room temperature, not only is the cycle-life characteristics of the battery improved, but the increase in resistance is also suppressed or reduced.

**Evaluation 2: Evaluation of High-temperature Storage Characteristics (Capacity Retention Rate/Capacity Recovery Rate/DC-IR Change Rate)**

**(1) Capacity Retention Rate and Capacity Recovery Rate**

Each of the rechargeable lithium battery cells according to Examples 1 to 9 and Comparative Examples 1 and 2 was once charged and discharged at 0.33 C to measure charge and discharge capacity (before the high-temperature storage).

Each of the rechargeable lithium battery cells of Examples 1 to 9 and Comparative Examples 1 and 2 was charged to SOC 100% (a state of being charged to 100% of charge capacity based on 100% of the entire charge capacity of the battery cell), stored at 60 °C for 60 days, and then, discharged to 3.0 V under a constant current condition at 0.33 C to measure initial discharge capacity.

The cells were each then recharged to 4.3 V at a constant current of 0.33 C and cut off at an ending current of 0.02 C and discharged to 3.0 V at the constant current of 0.33 C to twice measure discharge capacity. A ratio of first discharge capacity to the initial discharge capacity was calculated as a capacity retention rate (retention capacity), and

a ratio of the second discharge capacity to the initial discharge capacity was provided as a capacity recovery rate (recovery capacity).

**(2) DC-IR Change Rate**

Initial DC resistances (DC-IR) of the rechargeable lithium battery cells according to Examples 1 to 9 and Comparative Examples 1 and 2 were each measured with △V/△I (voltage change/current change), and DC resistances were measured after charging the cells into a full-charge state (SOC 100%) as an internal maximum energy state and storing them at a high temperature of 60 °C for 60 days (60D) to calculate DC-IR increase rates (%), and the results are shown in Table 3.

## Equation 3

$$\text{DC-IR increase rate} = (\text{DC-IR after 60 days} / \text{initial DC-IR}) * 100$$

(Table 2)

| | Capacity retention rate @60 °C, 60D(%) | Capacity recovery rate @60 °C, 60D(%) | Initial DC-IR (@25 °C, mΩ) | DC-IR @60 °C, 60D (mΩ) | DC-IR increase rate (%) |
|---|---|---|---|---|---|
| Example 1 | 76.7 | 79.1 | 21.20 | 29.61 | 133.4 |
| Example 2 | 81.9 | 84.2 | 21.76 | 27.85 | 128.0 |
| Example 3 | 73.0 | 76.8 | 22.07 | 29.15 | 132.1 |
| Example 4 | 73.4 | 76.0 | 23.11 | 31.38 | 135.8 |
| Example 5 | 72.5 | 76.4 | 23.96 | 32.15 | 134.2 |
| Example 6 | 77.7 | 82.9 | 22.21 | 28.32 | 127.5 |
| Example 7 | 83.1 | 86.7 | 22.34 | 29.11 | 130.3 |
| Example 8 | 79.2 | 81.2 | 22.22 | 28.75 | 129.4 |
| Example 9 | 75.5 | 79.7 | 23.08 | 29.40 | 127.4 |
| Comparative Example 1 | 61.0 | 73.7 | 21.00 | 31.94 | 152.1 |
| Comparative Example 2 | 65.9 | 74.0 | 22.90 | 33.16 | 144.8 |

Referring to Table 2, each of the rechargeable lithium battery cells of Examples 1 to 9, compared with the cells of Comparative Examples 1 and 2, exhibited improved capacity retention and an improved capacity recovery rate during the high-temperature storage and were also suppressed or reduced from an increase in resistance.

**Summary**

Referring to Tables 1 and 2, Examples 1 to 9 utilizing an cyclic acid anhydride-based compound (Chemical Formula 1) as an electrolyte additive as well as a cobalt-free positive electrode active material, compared with Comparative Example 1 utilizing no additive, improved storage characteristics at a high temperature as well as room temperature charging and discharging characteristics of the rechargeable lithium battery cells.

In contrast, in Comparative Example 2 utilizing a linear organic acid -based compound as an electrolyte additive as well as a cobalt-free positive electrode active material, the additive was reduced and decomposed on the surface of the negative electrode and thus formed an unstable film, which deteriorated a cycle-life of the rechargeable lithium battery cell, while increasing resistance. This phenomenon became more noticeable when Comparative Example 2 was stored at a high temperature.

In the present disclosure, the diameter (or size) of the particles may be measured utilizing a scanning electron microscope or a particle size analyzer. As the particle size analyzer, for example, HORIBA, LA-950 laser particle size analyzer, may be utilized. When the size of the particles is measured utilizing a particle size analyzer, the average particle diameter (or size) is referred to as D50. D50 refers to the average diameter (or size) of particles whose cumulative

volume corresponds to 50 vol% in the particle size distribution (e.g., cumulative distribution), and refers to the value of the particle size corresponding to 50% from the smallest particle when the total number of particles is 100% in the distribution curve accumulated in the order of the smallest particle size to the largest particle size. When particles are spherical, "diameter" indicates an average particle diameter, and when the particles are non-spherical, the "diameter" indicates a major axis length.

In the present disclosure, the term "comprise(s)/comprising", "include(s)/including", or "have(has)/having" are intended to designate that the performed characteristics, numbers, step, constituted elements, or a combination thereof is present, but it should be understood that the possibility of presence or addition of one or more other characteristics, numbers, steps, constituted element, or a combination are not to be precluded in advance.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

**Reference Numerals**

100: rechargeable lithium battery
10: positive electrode
20: negative electrode
30: separator
40: electrode assembly
50: case
60: sealing member
70: electrode tab

**Claims**

1. A rechargeable lithium battery (100) comprising

a positive electrode (10) comprising a cobalt-free positive electrode active material with a layered structure;
a negative electrode (20); and
an electrolyte comprising an additive represented by Chemical Formula 1:

Chemical Formula 1

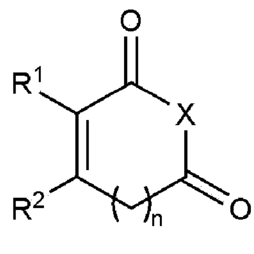

wherein, in Chemical Formula 1,

X is O or S;
$R^1$ and $R^2$ are each independently hydrogen, an unsubstituted C1 to C10 alkyl group, or are linked to each other to form a C6 to C20 cyclic hydrocarbon ring or aromatic ring; and
n is an integer from 0 to 3.

2. The rechargeable lithium battery (100) as claimed in claim 1, wherein,

the cobalt-free positive electrode active material having the layered structure comprises at least one kind of lithium composite oxide represented by Chemical Formula 2:

Chemical Formula 2 $Li_aNi_xMn_yM^1_zM^2_wO_{2\pm b}X_c$,

in Chemical Formula 2,

$0.5 \leq a < 1.8$, $0 \leq b \leq 0.1$, $0 \leq c \leq 0.1$, $0 \leq w < 0.1$, $0.6 \leq x < 1.0$, $0 < y < 0.4$, $0 < z < 0.1$, $w + x + y + z= 1$,
$M^1$ and $M^2$ being each independently one or more elements selected from among Al, Mg, Ti, Zr, Cr, Sr, V, B, W, Mo, Nb, Si, Ba, Ca, Ce, and Fe, and
X being one or more elements selected from among S, F, P, and Cl.

3. The rechargeable lithium battery (100) as claimed in claim 2, wherein, Chemical Formula 2 is represented by Chemical Formula 2-1:

Chemical Formula 2-1 $Li_aNi_{x1}Mn_{y1}Al_{z1}M^2_{w1}O_{2\pm b}X_c$,

in Chemical Formula 2-1,

$0.5 \leq a < 1.8$, $0 \leq b \leq 0.1$, $0 \leq c \leq 0.1$, $0 \leq w1 < 0.1$, $0.6 \leq x1 < 1.0$, $0 < y1 < 0.4$, $0 < z1 < 0.1$, $w1 + x1 + y1 + z1 = 1$,
$M^2$ being one or more elements selected from among Mg, Ti, Zr, Cr, Sr, V, B, W, Mo, Nb, Si, Ba, Ca, Ce, and Fe, and
X being one or more elements selected from among S, F, P, and Cl.

4. The rechargeable lithium battery (100) as claimed in any one of the preceding claims, wherein in Chemical Formula 1, XisO.

5. The rechargeable lithium battery (100) as claimed in any one of the preceding claims, wherein in Chemical Formula 1, $R^1$ and $R^2$ are each independently hydrogen or a methyl group, or are linked to each other to form a cyclohexane ring.

6. The rechargeable lithium battery (100) as claimed in any one of the preceding claims, wherein in Chemical Formula 1, n is 0 or 1.

7. The rechargeable lithium battery (100) as claimed in claim 1, wherein, Chemical Formula 1 is one selected from among Chemical Formulas 1-1 to 1-5:

Chemical Formula 1-1

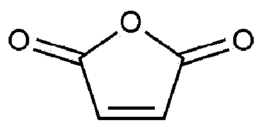

Chemical Formula 1-2

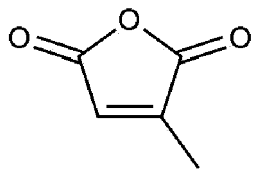

Chemical Formula 1-3

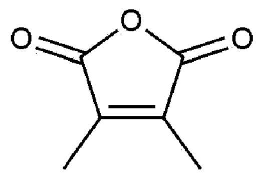

Chemical Formula 1-4

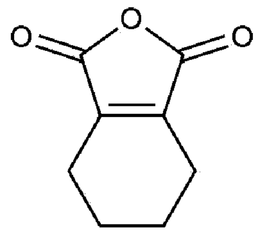

Chemical Formula 1-5

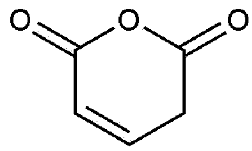

.

**8.** The rechargeable lithium battery (100) as claimed in any one of the preceding claims, wherein, the additive is in an amount of 0.05 to 3 wt% based on a total weight, 100 wt%, of the electrolyte.

**9.** The rechargeable lithium battery (100) as claimed in any one of the preceding claims, wherein, the electrolyte further comprises a non-aqueous organic solvent and a lithium salt.

**10.** The rechargeable lithium battery (100) as claimed in any one of the preceding claims, wherein, the rechargeable lithium battery (100) further comprises a separator (30) between the positive electrode (10) and the negative electrode (20).

【FIG. 1】

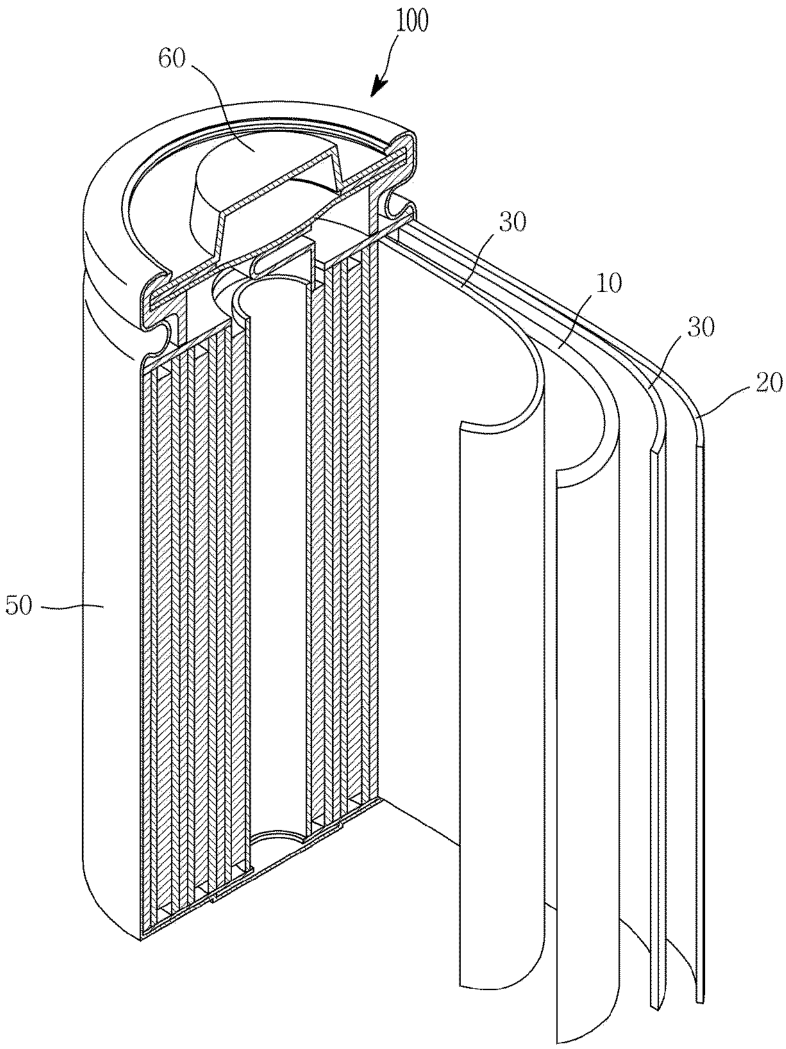

【FIG. 2】

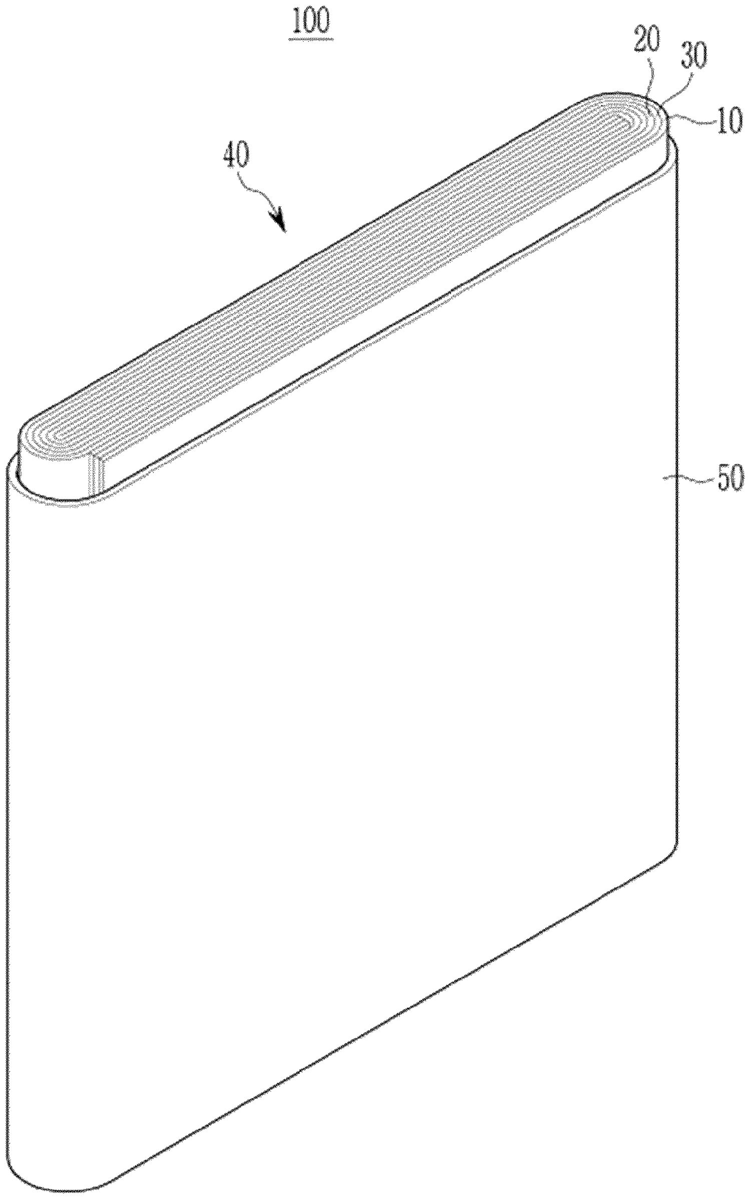

【FIG. 3】

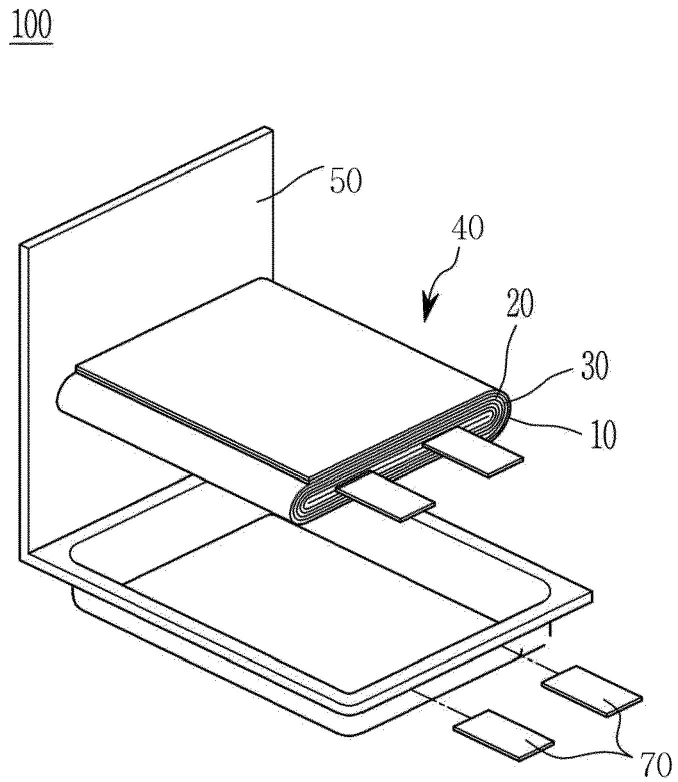

Europäisches Patentamt
European Patent Office
Office européen des brevets

# EUROPEAN SEARCH REPORT

Application Number
EP 24 17 9930

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 377 497 A (SVOLT ENERGY TECH CO LTD) 22 November 2022 (2022-11-22)<br>* claims 1,2,7,8 *<br>----- | 1-10 | INV.<br>H01M4/13<br>H01M10/0525<br>H01M10/0567 |
| X | EP 3 340 360 A1 (TOYOTA MOTOR CO LTD [JP]; DAIKIN IND LTD [JP])<br>27 June 2018 (2018-06-27)<br>* claims 1,5,9 *<br>* paragraph [0067] *<br>----- | 1-10 | |
| | | | TECHNICAL FIELDS SEARCHED (IPC) |
| | | | H01M |

The present search report has been drawn up for all claims

1

| Place of search | Date of completion of the search | Examiner |
|---|---|---|
| The Hague | 18 October 2024 | Domínguez Gutiérrez |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 17 9930

This annex lists the patent family members relating to the patent documents cited in the above-mentioned European search report. The members are as contained in the European Patent Office EDP file on
The European Patent Office is in no way liable for these particulars which are merely given for the purpose of information.

18-10-2024

| Patent document cited in search report | Publication date | Patent family member(s) | Publication date |
|---|---|---|---|
| CN 115377497 A | 22-11-2022 | NONE | |
| EP 3340360 A1 | 27-06-2018 | BR 102017026415 A2 | 17-07-2018 |
| | | CN 108242570 A | 03-07-2018 |
| | | EP 3340360 A1 | 27-06-2018 |
| | | JP 6700166 B2 | 27-05-2020 |
| | | JP 2018106896 A | 05-07-2018 |
| | | KR 20180075405 A | 04-07-2018 |
| | | KR 20190133131 A | 02-12-2019 |
| | | RU 2682323 C1 | 19-03-2019 |
| | | US 2018183105 A1 | 28-06-2018 |

EPO FORM P0459

For more details about this annex : see Official Journal of the European Patent Office, No. 12/82